# EUROPÄISCHE PATENTSCHRIFT

(11) **EP 0 407 650 B1**
(45) Veröffentlichungstag und Bekanntmachung des Hinweises auf die Patenterteilung: **06.10.1993**
(21) Anmeldenummer: 89113297.9
(22) Anmeldetag: 20.07.1989
(51) Int. Cl.: A61F 2/66

(54) **Kunstfuss für eine Beinprothese**
Artificial foot for a leg prothesis
Pied artificiel pour une prothèse de jambe

(30) Priorität: 08.07.1989 DE 8908356 U
(43) Veröffentlichungstag der Anmeldung: 16.01.1991
(73) Patentinhaber: IPOS GmbH & CO. KG., D-21337 Lüneburg (DE)
(72) Erfinder: Prahl, Gregor M., D-2127 Rullstorf (DE)
(74) Vertreter: Richter, Werdermann & Gerbaulet

(56) Entgegenhaltungen:
- EP-A- 0 234 886
- WO-A-88/00815
- DE-C- 807 214
- DE-U- 8 804 228
- US-A- 3 484 871
- US-A- 4 547 913

## Beschreibung

Die Erfindung betrifft einen Kunstfuß für eine Beinprothese gemäß dem Oberbegriff des Anspruch 1.

Ein derartiger Kunstfuß ist durch die DE-U-88 04 228 bekannt. Bei diesem Kunstfuß besteht der Versteifungskörper aus mindestens zwei etwa gleich langen, übereinanderliegenden Blattfedern, zwischen denen ein Abstandshalter angeordnet ist. Die beiden Blattfedern und der Abstandshalter sind entsprechend dem Abrollprofil des Fußes geformt und im Ballenbereich mit einer fußballengleich verlaufenden Kröpfung versehen; im Fußmittelbereich sind die Blattfedern und der Abstandshalter parallel und geradlinig verlaufend, wohingegen ihre der Fußspitze zugekehrten Enden in etwa parallel zueinander verlaufende Kröpfungen auslaufen. Dieser Kunstfuß soll aufgrund seiner Ausgestaltung eine große Beweglichkeit im Zehengrundgelenk aufweisen, sowie eine elastische, nachgiebige Abfederung sowohl im Vorderfuß als auch im Fersenbereich, wobei der Abrollwiderstand für einen längeren Zeitraum unveränderlich vorgegeben ist. Durch diese Abstützung des metallischen Versteifungskörpers im Fersenteil des Fußformteils wird eine erhöhte Festigkeit des Fußformteils erreicht, ohne daß die erforderlichen elastischen und nachgiebigen Abfederungen im Vorderfuß und im Fersenbereich beeinträchtigt werden. Insbesondere soll diese bekannte Konstruktion ermöglichen, daß durch die federnd gestalteten Vorder- und Mittelfußbereiche Bewegungsenergie aufgenommen, gespeichert und in der Entlastung der Schrittrücklage wieder als frei werdende Energie in die Prothese eingespeist wird. Diese Rückführung frei gewordener Bewegungsenergie in die prothetischen Systeme führt zu einer Energieentlastung während der Fortbewegung des Patienten. Trotz dieser Vorteile ist eine für den Patienten spürbare Elastizität im Vorderfußbereich nicht gegeben, was darauf zurückzuführen ist, daß die beiden Blattfedern und der Abstandshalter durch die vorderseitigen fußballengleich verlaufenden Kröpfungen im Ballenbereich eine gewisse Steifigkeit aufweisen, was bedingt ist durch die sandwichartige Ausgestaltung und Anordnung der beiden Blattfedern und der zwischen diesen angeordneten Abstandshaltern. Außerdem kommt es leicht zu Horizontalverschiebungen zwischen den metallischen Blattfedern, so daß die in das Prothesensystem bei der Rückfederung zurückfließende Energie gering ist. Ferner weist die bekannte Konstruktion ein hohes Gewicht auf.

Als Material für Kunstfüße wird seit langem Polyurethan-Schaumkunststoff eingesetzt, der den Vorteil eines niedrigen Gewichtes aufweist.

Aus der DE-A-354 246 ist ferner ein Kunstfuß bekannt, bei dem im Sohlenbereich des Kunstfußes eine Metallschiene eingebettet ist. Dieser künstliche Fuß soll das Weitausschreiten eines Fuß- oder Beinamputierten ermöglichen, da das Weitausschreiten das Schrägnachhintenverlegen des Unterschenkels, also das Nachgeben der Ferse des mit seiner ganzen Unterfläche auf dem Boden aufruhenden Fußes bedingt. Er soll ferner das leichte Wiederaufrichten des Unterschenkels bedingen, ohne daß dieser die Senkrechtlage überschreitet, während das Verlegen des Körpergewichtes nach vorn und das Anheben der Ferse durch die übliche Nachgiebigkeit des Mittelfuß- und Zehenteils des Kunstfußes ermöglicht werden soll. Diese Nachgiebigkeit soll durch Einlagern einer durch den Sohlen- und Fersenteil hindurchgehenden Federplatte erzielt werden. Um nunmehr die Ausladung des Unterschenkels schräg nach hinten herbeiführen zu können, sieht diese bekannte Ausführungsform eines Kunstfußes eine Verbindung zwischen dem starren Unterschenkel und der Federplatte vor, bei der sich der Schenkel in Art einer Wiege aus seiner Aufrechtstellung nach rückwärts bewegt und bei Verlegung des Körpergewichtes nach vorne wieder in die erste übergeht. Diese Schaukelbewegung erfolgt dadurch, daß der keilförmig zulaufende, starre Unterschenkel in einem Sattel sitzt, dessen eine Schräge vom Spann des Kunstfußes und dessen Gegenschräge von einer Abzweigung der Federplatte gebildet wird. Zur weiteren Abfederung befindet sich zwischen der Abzweigung und der eigentlichen Federplatte ein zusammendrückbares Keilkissen, z.B. aus weichem Gummi. Damit beim Anstoßen der vorderen Keilfläche am Schenkel gegen den Fuß kein unerwünschtes Geräusch auftritt, besteht der Vorder- bzw. Mittelfuß aus einer schmiegsamen oder leicht zusammendrückbaren, jedenfalls geräuschdämpfenden Masse, vorzugsweise Filz. Mit einer Kappe aus diesem Stoff ist auch die Widerlagerfläche des Unterschenkelks überzogen. Die Abzweigung der Federplatte ist bei diesem künstlichen Fuß mit der hinteren Keilfläche des Unterschenkels fest verbunden. Dabei wird jedoch die Abzweigung nicht scharf abgebogen, da eine wiegende oder schaukelnde Bewegung angestrebt wird. Sie ist daher mit ihrem Knie gleitend auf der Platte angebracht.

Bei diesem bekannten Kunstfuß ist somit eine Stahlfeder mit federnden Eigenschaften eingesetzt, die bis in den Vorfuß reicht. Die wichtigen Elastizitätsunterschiede zwischen Mittelfuß und dem Vorfuß sind hier jedoch konstruktiv nicht berücksichtigt. Die verwendete Stahlfeder ermöglicht jedoch kein natürliches Abrollen, auch sind die Verbindungsprobleme zwischen den verschiedenen Materialien im elastischen Bereich nicht konstruktiv gelöst.

Die DE-A-361 972 beschreibt einen künstlichen Fuß mit einer aus mehreren, gegeneinander abgestuften Blattfedern bestehenden Längsfederung, deren Blattfedern mit ihren rückwärtigen Enden auf der unteren Seite eines den hinteren Fußteil bildenden, aber von der Sohle durch eine Zwischenschicht getrennten starren Klotzes befestigt sind, während die vorderen in geeigneter doppelter Krümmung abwärts geführten Federenden unmittelbar auf die zweckmäßig durch ein Schutzblech bedeckte Sohle aufdrücken. Auch bei diesem künstlichen Fuß wird versucht, die Fußbewegungen über eine Metallfeder zu steuern; sie läßt jedoch keine Beweglichkeit im Zehengrundbereich zu.

Bei einem aus der US-A-2,556,525 bekannten Kunstfuß für Beinprothesen ist in einem äußeren Schaumkunststoff-Fußformteil ein steifer, jedoch flexibler Kunststoffteil eingelassen, der sich über die gesamte Länge des Fußes erstreckt, wobei in diesem steifen, jedoch flexiblen Kunststoffteil eine Metalleinlage aus Federstahl eingebettet ist. Auch wenn ein innerer, teilweise flexibler Kunststoffteil mit einer Metalleinlage aus Federstahl bei diesem bekannten Kunstfuß verwendet wird, so ist es bei diesem Kunstfuß nicht möglich, die Abrollfunktion auf die von der Natur vorgegebene Drittelungslinie zu legen. Im übrigen ist auch die Metalleinlage bis in die Fußspitze geführt. Trotz der Verwendung eines flexiblen Kunststoffteiles und einer Metalleinlage aus Federstahl ist eine ausreichende Beweglichkeit im Zehengrundgelenk, wie dies in der Natur gegeben ist, nicht möglich. Das wichtige Einknicken im Zehengrundgelenk ist bei dieser bekannten Ausführungsform nicht berücksichtigt und aufgrund der konstruktiven Ausgestaltung auch nicht möglich.

Um einen Kunstfuß für Beinprothesen mit einem über einen längeren Zeitraum vorgegebenen Abrollwiderstand hoher Elastizität und einer Einknickmöglichkeit im Zehengrundgelenk zu schaffen, ist daher in der DE-A-23 41 887 ein weiterer Kunstfuß vorgeschlagen worden. Bei diesem Kunstfuß wird die Vorfußelastizität durch Einsatz eines homogenen Vulkollanteils (Elastomer) gewährleistet, d.h., es wird im Vorfuß eine Rückstellelastizität berücksichtigt, die weit über bis dahin geformte Polstereffekte hinausging. Allerdings war man bisher nicht in der Lage, die Vorteile dieser Rückstellelastizität in ihrer physikalischen Einflußgröße auf das Gangbild funktionell zu nutzen.

Es ist Aufgabe der vorliegenden Erfindung, den eingangs genannten Kunstfuß dahingehend zu verbessern, daß bei einem Abrollmoment bis zu 140 Nm auch bei Dauerbeanspruchung (Lastwechselzahl bis zu 10 Millionen) eine mindestens gleichbleibende Mittelfußelastizität vorzugsweise bei verringertem Gewicht erzielt wird, daß Horizontalverschiebungen zwischen den metallischen Blattfedern im Vorderfußbereich vermieden werden und daß im Vorderfußbereich eine hohe Elastizität gegeben ist.

Diese Aufgabe wird durch die im Anspruch 1 aufgeführte Merkmalskombination gelöst.

Die verwendete kohlefaserverstärkte Kunstharz-Blattfeder hat auf dem Prüfstand sehr viel günstigere Lastwechselzahlen erbracht. Insbesondere durch die viel kürzer ausgebildeten oberen und unteren metallischen Blattfedern wird in erheblichem Umfang das Gewicht des Kunstfußes reduziert. Des weiteren wird der Vorteil erreicht, daß Horizontalverschiebungen zwischen den metallischen Blattfedern im Vorderfußbereich, nicht mehr auftreten können. Darüber hinaus läßt sich mit der vorliegenden Konstruktion eine hohe Gewichtsersparnis erreichen. Durch den Abstand k zwischen der durch die hintere Blattfederebene und der Kröpfung bestimmten Ebene werden relativ große Federwege erreicht, die zum Teil den Einsatz von Gelenken durch ihre funktionell große Dorsalflexion überflüssig machen. Gleichzeitig entsteht bei der Rückfederung ein großer Bereich frei werdender Energie, die in das Prothesensystem wieder eingespeist werden kann. Die Blattfederkonstruktion ist bei minimalem Gewicht einer hohen Abrollbelastung auf Dauer gewachsen; sie arbeitet völlig geräuschfrei und zeichnet sich durch große Federwege aus. Aufgrund der Kombination der Titan-Blattfedern und der kohlefaserverstärkten Kunstharz-Blattfeder in Verbindung mit den Gleiteinsätzen ist die Erfindung anderen, nach dem Stand der Technik bekannten Kunstfußkonstruktionen überlegen.

Weitere vorteilhafte Ausführungsformen gehen aus den Unteransprüchen hervor. So ist u.a. bei einer bevorzugten Ausführungsform der federnde Teil des Kunstfußes folgendermaßen aufgebaut: Die kohlefaserverstärkte Kunstharz-Blattfeder erstreckt sich bis in den Fußballenbereich und liegt zwischen einer oberen Feder aus Titan, die in der Fußmitte endet, und einer unteren Blattfeder aus Titan, die wesentlich kürzer als die obere Feder ausgestattet ist. Zwischen den Federn ist jeweils ein Gleiteinsatz aus Polyäthylen angeordnet. Dieser federnde Teil wird, wie im Prinzip nach dem Stand der Technik bekannt, in den Fuß einvulkanisiert. Bei dem dargelegten Federaufbau liegt die Gewichtsreduzierung gegenüber dem bekannten Stand der Technik bei etwa 40%.

Ein Ausführungsbeispiel der Erfindung soll anhand der Zeichnungen im nachfolgenden erläutert werden. Es zeigen
Fig. 1 einen Schnitt durch den Kunstfuß,
Fig. 2 eine Seitenansicht des Versteifungskörpers, und
Fig. 3 eine Draufsicht auf die kohlefaserverstärkte Kunstharz-Blattfeder mit aufgelegtem, elastischem Gleiteinsatz.

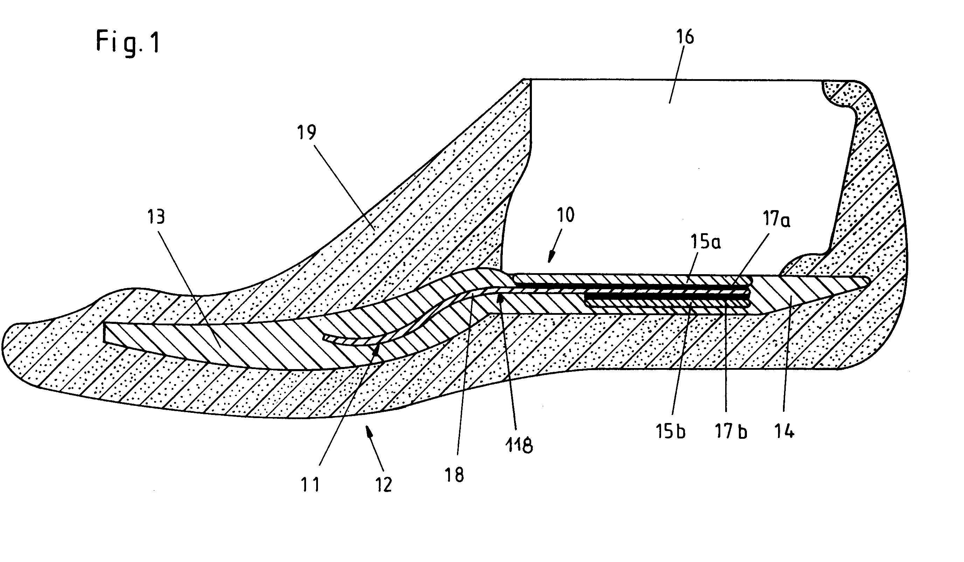

Der in Fig. 1 dargestellte Kunstfuß besitzt einen Versteifungskörper 10 mit einer nach unten gerichteten Kröpfung 11 im Ballenbereich 12, die dem Abrollprofil des Kunstfußes entsprechend geformt ist. Der Versteifungskörper 10 ist in einem Vorfußkern 13 ebenso eingebettet wie in einer im Fersenbereich liegenden Lasche 14. Der Versteifungskörper 10 teilt sich in eine obere Blattfeder 15a, einen Gleiteinsatz 17a aus Polyäthylen, eine kohlefaserverstärkte Kunststoff-Blattfeder 18 als Abstandshalter 118, die bis in den Fußballenbereich reicht und am vorderen Ende gekröpft ist, einen weiteren Gleiteinsatz 17b sowie einer den Versteifungskörper nach unten abschließenden unteren Titan-Feder 15b auf. Der aus dem Versteifungskörper 10, dem Vorfußkern 13 und der Lasche 14 bestehende Funktionskern ist von einem Schaumkunststoffußformteil 19 umschäumt, das im rückwärtigen Teil einen Hohlraum 16 aufweist, der bis auf den Versteifungskörper 10, d.h. die obere Titan-Blattfeder 15a, geführt ist und in dem Gelenke oder starre Befestigungselemente direkt an dem Versteifungskörper 10 verschraubt werden können.

Die nach unten gerichtete Kröpfung 11 ist dergestalt ausgebildet, daß die Abrollfunktion auf die von der Natur vorgegebene vordere Drittelungslinie (Zehengrundgelenklinie) gelegt ist. Dabei ist an der Kröpfung 11 des Versteifungskörpers 10 ein aus einem Elastomer bestehender Vorfußkern 13 anvulkanisiert, der in seiner unteren Abschlußlinie der äußeren Form des Fußballens entspricht. Dieser Vorfußkern ist durch eine umschäumte Struktur völlig alterungsbeständig, so daß der Abrollwiderstand über längere Zeiträume unveränderlich vorbestimmbar ist. Gleichfalls ist im Fersenteil zur Verstärkung des Versteifungskörpers 10 ebenfalls aus einem Elastomer eine Lasche 14 anvulkanisiert.

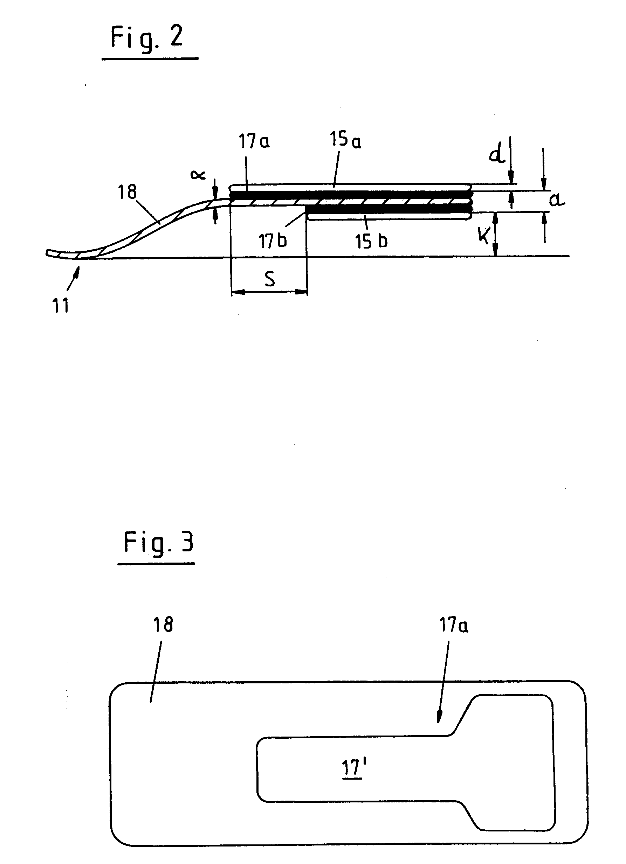

Der der Erfindung insbesondere betreffende Aufbau des Versteifungskörpers 10 ist aus Fig.2 und Fig.3 im Detail ersichtlich. Die Mittelfußkonstruktion besteht hierbei aus einer oberen, etwa bis in die Fußmitte reichenden Titan-Blattfeder 15a, einer kohlefaserverstärkten Kunstharz-Blattfeder 18, die am vorderen Ende gekröpft ist und etwa bis in den Fußballenbereich hineinragt, und einer unteren Blattfeder 15b. Zwischen den Blattfedern 15a und 15b und der kohlefaserverstärkten Kunstharz-Blattfeder 18 sind Gleiteinsätze 17a und 17b angeordnet. Die obere metallische Blattfeder 15a überragt die untere metallische Blattfeder 15b um das Maß s, das mindestens 15 mm beträgt. Wie aus Fig. 3 ersichtlich, ist insbesondere die Blattfeder 18 im wesentlichen rechteckig ausgebildet, wobei der Abstandhalter 17a etwa eine T-Form besitzt, deren zungenförmiger Schenkel bis zur Mitte des Fußes reicht. Dieser Gleiteinsatz verhindert einen Kontakt der Kunstharz-Blattfeder 18 mit der oberen Titan-Blattfeder 15a. Die Gleiteinsätze 17a und 17b sind gleichzeitig geräuschdämmend und erlauben eine Steuerung der maximal aufzunehmenden Federkraft. Je größer der Abstand a zwischen den metallischen Blattfedern bzw. zwischen je einer Blattfeder aus Titan und einer Kunstharz-Blattfeder 18 ist, desto größer ist die maximale Belastbarkeit des Kunstfußes. Es ist jedoch ebenso möglich, Gleiteinsätze 17a und 17b aus Polyurethan, insbesondere einem vernetzten Polyurethan-Elastomer, zu verwenden. Polyurethan weist eine hohe Abriebfestigkeit auf, so daß immer ein gleichbleibender Abrolleffekt gewährleistet ist. Hinzu kommt das hohe, selbständige Rückstellvermögen aus einer verformten Stellung in die Ausgangsstellung. Auch der Fußkern 13 und die Lasche 14 bestehen aus Polyurethan, insbesondere einem vernetzten Polyurethan-Elastomer, das unter dem Handelsnamen Vulkollan bekannt ist. Auch Polyurethane, die durch Umsetzen von Diisocyanaten, die unter dem Handelsnamen Desmodur bekannt sind, nach dem Polyisocyanat-Polyadditionsverfahren erhalten werden, können eingesetzt werden. Dabei häufig eingesetzte Isocyanat-Typen sind TDI, MDI und HDI. Für stark vernetzte Polyurethane kommen vor allem Tri- und u.a. auch Polyisocyanate zum Einsatz. Auch hier wird die Eigenschaft einer hohen Gleitfähigkeit ausgenutzt.

## Patentansprüche

1. Kunstfuß für eine Beinprothese aus einem Schaumkunststoff-Fußformteil mit einem aus einem in seinem Sohlenbereich eingebetteten, plattenförmigen Versteifungskörper (10), der mindestens zwei übereinanderliegende Blattfedern (15a,15b) mit einem zwischen diesen angeordneten, elastischen Abstandshalter (118) aufweist und der im Ballenbereich (12) mit einer fußballengleich verlaufenden Kröpfung (11) zur Unterstützung der Fußabrollfunktion ausgebildet ist, wobei der Abstandhalter (118) dem Abrollprofil des Fußes entsprechend geformt ist und sich bis in dem Bereich der Kröpfung (11) erstreckt, und mit einem als elastisch nachgiebige Abfederung dienenden Vorfußkern (13) und einer im Fersenbereich angeordneten Lasche (14) bestehenden Funktionskern, wobei der Funktionskern und der Versteifungskörper (10) fest miteinander verbunden sind, dadurch gekennzeichnet, daß der Abstandshalter (118) aus einer kohlefaserverstärkten Kunstharz-Blattfeder (18) besteht, daß die obere (15a) und die untere Blattfeder (15b) eben ausgebildet sind, wobei das zur Fußspitze weisende Ende der oberen Blattfeder (15a) etwa bis zur Fußmitte reichend ist, daß die untere Blattfeder (15b) gegenüber der oberen Blattfeder (15a) kürzer bemessen ausgebildet ist, und daß jeweils zwischen den Blattfedern (15a,15b) und der kohlefaserverstärkten Kunstharz-Blattfeder (18) ein elastischer Gleiteinsatz (17a,17b) aus Polyäthylen mit einer hohen Gleitfähigkeit angeordnet ist.

2. Kunstfuß nach Anspruch 1, dadurch gekennzeichnet, daß die untere und die obere Blattfeder (15a,15b) im hinteren Bereich etwa in der gleichen Vertikalebene enden.

3. Kunstfuß nach einem der Ansprüche 1 und 2, dadurch gekennzeichnet, daß die Blattfedern (15a,15b) aus einem metallischen Werkstoff bestehen.

4. Kunstfuß nach einem der Ansprüche 1 bis 3, dadurch gekennzeichnet, daß die Blattfedern (15a,15b) aus Titan, einer hochfesten Ti-Al-Legierung od.dgl. bestehen.

5. Kunstfuß nach einem der Ansprüche 1 bis 4, dadurch gekennzeichnet, daß mindestens zwei der Blattfedern (15a,15b,18) gleich dick sind, vorzugsweise eine Dicke (d) von 2,7 mm aufweisen.

6. Kunstfuß nach einem der Ansprüche 1 bis 5, dadurch gekennzeichnet, daß die obere Blattfeder (15a) die untere (15b) zur Fußspitze hin um mindestens 15 mm (Strecke s) überragt.

7. Kunstfuß nach einem der Ansprüche 1 bis 6, dadurch gekennzeichnet, daß der Versteifungskörper (10) bzw. die Blattfedern (15a,15b) und/oder die kohlefaserverstärkte Kunstharz-Blattfeder (18) in einer Draufsichtprojektion im wesentlichen rechteckig sind, d.h. abgerundete Ecken und Kanten besitzen.

8. Kunstfuß nach einem der Ansprüche 1 bis 7, dadurch gekennzeichnet, daß die Gleiteinsätze (17a,17b) zwischen den Blattfedern (15a,15b) und der kohlefaserverstärkten Kunstharz-Blattfeder (18) aus eine hohe Gleitfähigkeit aufweisendem, hochmolekularem Polyäthylen oder einem anderen geeigneten Werkstoff bestehen.

9. Kunstfuß nach einem der Ansprüche 1 bis 8, dadurch gekennzeichnet, daß der Abstand (a) der Blattfedern (15a,15b) im Fersenbereich mindestens 3 mm beträgt.

10. Kunstfuß nach Anspruch 8 oder 9, dadurch gekennzeichnet, daß die Gleiteinsätze (17a,17b) 1 mm dick sind.

11. Kunstfuß nach einem der Ansprüche 8 bis 10, dadurch gekennzeichnet, daß die Gleiteinsätze (17a,17b) im Fersenbereich im wesentlichen T-förmig und der zur Fußspitze hin gerichtete Schenkel (17') zungenförmig ausgebildet sind, vorzugsweise dergestalt, daß die Blattfedern (15a,15b) und/oder die kohlefaserverstärkte Kunstharz-Blattfeder (18) die Gleiteinsätze an allen Seiten überragen.

12. Kunstfuß nach einem der Ansprüche 1 bis 11, dadurch gekennzeichnet, daß die Blattfedern (15a,15b) und/oder die kohlefaserverstärkte Kunstharz-Blattfeder (18) in den Fußkern (13) und die Lasche (14) eingebettet sind, die beide aus Polyurethan, insbesondere einem vernetzten Polyurethan-Elastomer bestehen.

13. Kunstfuß nach einem der Ansprüche 1 bis 8, dadurch gekennzeichnet, daß die kohlefaserverstärkte Kunstharz-Blattfeder (18) vom Fersenbereich bis in den Mittelfußbereich im wesentlichen eben ausgebildet ist und die Kröpfung (11) im Ballenbereich (12) mindestens 15 mm, vorzugsweise 16 bis 21 mm, tiefer verläuft (Abstand k).

14. Kunstfuß nach einem der Ansprüche 1 bis 13, dadurch gekennzeichnet, daß die kohlefaserverstärkte Kunstharz-Blattfeder (18) im Bereich der Kröpfung (11) mindestens so breit wie im Fersenbereich ausgebildet ist.

## Claims

1. Artificial foot for a leg prosthesis of a foamed plastic molded foot portion having a plate-like reinforcing member (10) comprising a function core embedded within its sole area, which is provided with at least two superjacently arranged leaf springs (15a,15b) with an elastic spacing means (118) disposed therebetween and which, within the area of the ball of the foot (12), is constructed with a bend (11) proceeding in a ball of the foot-like manner so as to assist the rolling function of the foot, in which the spacing means (118) is configured so as to correspond to the rolling contour of the foot and so as to extend into the area of the bend (11), and with a fore-foot core (13) serving as a resiliently yielding shock absorber and a tongue (14) disposed within the heel area, in which case the function core and the reinforcing member (10) are rigidly interconnected, characterized in that the spacing means (118) comprises a carbon fiber-reinforced synthetic resin leaf spring (18), in that the top (15a) and the bottom leaf spring (15b) are constructed so as to be plane, in which the end of the top leaf spring (15a) which points toward the tip of the foot reaches to approximately to the center of the foot, in that the bottom leaf spring (15b), in comparison with the top leaf spring (15a), is constructed so as to be shorter, and in that, in each case, between the leaf springs (15a, 15b) and the carbon fiber-reinforced synthetic resin leaf spring (18), an elastic gliding insert (17a,17b) of polyethylene possessing a high gliding ability is disposed.

2. Artificial foot according to Claim 1, characterized in that the bottom and the top leaf spring (15a,15b), within the rear area, terminate approximately in the same plane.

3. Artificial foot according to either Claim 1 or 2, characterized in that the leaf springs (15a,15b) are comprised of a metallic material.

4. Artificial foot according to any of Claims 1 to 3, characterized in that the leaf springs (15a,15b) are comprised of titanium, of a high-tensile Ti-Al alloy or suchlike.

5. Artificial foot according to any of Claims 1 to 4, characterized in that at least two of the leaf springs (15a, 15b) are of an identical thickness and preferably possess a thickness (d) of 2.7 mm.

6. Artificial foot according to any of Claims 1 to 5, characterized in that the top leaf spring (15a) projects over the bottom leaf spring (15b) in the direction of the foot by at least 15 mm (path s).

7. Artificial foot according to any of Claims 1 to 6, characterized in that the reinforcing member (10) or the leaf springs (15a,15b) and/or the carbon fiber-reinforced synthetic resin leaf spring (18), in a top view projection, are substantially rectangular, i.e. have rounded-off corners and edges.

8. Artificial foot according to any of Claims 1 to 7, characterized in that the gliding inserts (17a,17b) and the carbon fiber-reinforced synthetic resin leaf spring (18) are comprised of a high-molecular polyethylene possessing a high gliding ability or of some other suitable material.

9. Artificial foot according to any of Claims 1 to 8, characterized in that the distance (a) of the leaf springs (15a,15b) within the heel area is at least 3 mm.

10. Artificial foot according to Claim 8 or 9, characterized in that the gliding inserts (17a,17b) have a thickness of 1 mm.

11. Artificial foot according to any of Claims 8 to 10, characterized in that the gliding inserts (17a,17b) are, within the heel area, substantially T-shaped and that the leg (17') directed toward the tip of the foot is constructed so as to be tongue-shaped, by preference in such a way that the leaf springs (15a,15b) and/or the carbon fiber-reinforced synthetic resin leaf spring (18) project over the gliding inserts on all sides.

12. Artificial foot according to any of Claims 1 to 11, characterized in that the leaf springs (15a,15b) and/or the carbon fiber-reinforced synthetic resin leaf spring (18) are embedded in the foot core (13) and the tongue (14) are both comprised of polyurethane, more particularly a crosslinked polyurethane elastomer.

13. Artificial foot according to any of Claims 1 to 8, characterized in that the carbon fiber-reinforced synthetic resin leaf spring (18), from the heel area into the metatarsus area, is substantially constructed so as to be plane and in that the bend (11), within the area of the ball of the foot (12), proceeds at least 15 mm, preferably 16 to 21 mm, deeper (distance k).

14. Artificial foot according to any of Claims 1 to 13, characterized in that the carbon fiber-reinforced synthetic resin leaf spring (18), within the area of the bend (11), is constructed so as to be at least as wide as within the heel area.

## Revendications

1. Pied artificiel pour une prothèse de jambe constitué par une pièce moulée en pied en matière plastique mousse avec un corps de renforcement (10) en forme de plaque, encastré dans sa partie semelle, qui présente au moins deux ressorts à lame (15a, 15b) superposés avec un écarteur (118) élastique, placé entre ceux-ci et qui est configuré dans la zone de la cambrure (12) avec une coudure (11) qui a une allure semblable à celle d'une cambrure de pied pour soutenir la fonction de déroulement du pied, l'écarteur (118) étant formé de manière à correspondre au profil de déroulement du pied et s'étendant jusque dans la zone de la coudure (11), et avec un noyau de fonction constitué par un noyau de pied avant (13) qui sert de suspension sur ressort souple élastique et une languette (14) placée dans la partie talon, le noyau de fonction et le corps de renforcement (10) étant reliés de manière solide l'un à l'autre, **caractérisé en ce** que l'écarteur (118) est constitué par un ressort à lame (18) en résine synthétique renforcée par fibres de carbone, que le ressort à lame supérieur (15a) et le ressort à lame inférieur (15b) sont configurés en étant plats, l'extrémité du ressort à lame supérieur (15a) qui est orientée vers la pointe du pied allant jusqu'à peu près le milieu du pied, que le ressort à lame inférieur (15b) est configuré en étant plus court que le ressort à lame supérieur (15a) et qu'un insert coulissant élastique (17a, 17b) en polyéthylène avec un grand pouvoir coulissant est placé respectivement entre les ressorts à lame (15a, 15b) et le ressort à lame en résine synthétique renforcé par fibres de carbone (18).

2. Pied artificiel selon la revendication 1, **caractérisé en ce** que le ressort à lame inférieur et le ressort à lame supérieur (15a, 15b) se terminent à peu près dans le même plan vertical dans la zone postérieure.

3. Pied artificiel selon l'une des revendications 1 et 2, **caractérisé en ce** que les ressorts à lame (15a, 15b) sont en un matériau métallique.

4. Pied artificiel selon l'une des revendications 1 à 3, **caractérisé en ce** que les ressorts à lame (15a, 15b) sont constitués en titan, en un alliage à haute résistance de titan et d'aluminium ou équivalent.

5. Pied artificiel selon l'une des revendications 1 à 4, **caractérisé en ce** qu'au moins deux des ressorts à lame (15a, 15b, 18) sont de même épaisseur et qu'ils ont de préférence une épaisseur (d) de 2,7 mm.

6. Pied artificiel selon l'une des revendications 1 à 5, **caractérisé en ce** que le ressort à lame supérieur (15a) dépasse le ressort à lame inférieur (15b) jusqu'à la pointe du pied d'au moins 15 mm (distance s).

7. Pied artificiel selon l'une des revendications 1 à 6, **caractérisé en ce** que le corps de renforcement (10) ou les ressorts à lame (15a, 15b) et/ou le ressort à lame en résine synthétique renforcée par fibres de carbone (18) sont substantiellement rectangulaires en projection en vue de dessus, c'est-à-dire qu'ils possèdent des coins et des bords arrondis.

8. Pied artificiel selon l'une des revendications 1 à 7, **caractérisé en ce** que les inserts coulissants (17a, 17b) entre les ressorts à lame (15a, 15b) et le ressort à lame en résine synthétique renforcée par fibres de carbone (18) sont constitués par du polyéthylène à haute densité moléculaire qui présente un pouvoir coulissant élevé ou en une autre matière appropriée.

9. Pied artificiel selon l'une des revendications 1 à 8, **caractérisé en ce** que l'écart (a) entre les ressorts à lame (15a, 15b) est d'au moins 3 mm dans la zone du talon.

10. Pied artificiel selon la revendication 8 ou 9, **caractérisé en ce** que les inserts coulissants (17a, 17b) ont 1 mm d'épaisseur.

11. Pied artificiel selon l'une des revendications 8 à 10, **caractérisé en ce** que les inserts coulissants (17a, 17b) sont configurés dans la zone du talon en étant substantiellement en forme de T et le montant (17') dirigé vers la pointe du pied est configuré en forme de languette, de préférence de manière telle que les ressorts à lame (15a, 15b) et/ou le ressort à lame en résine synthétique renforcée par fibres de carbone (18) dépassent les inserts coulissants de tous les côtés.

12. Pied artificiel selon l'une des revendications 1 à 11, **caractérisé en ce** que les ressorts à lame (15a, 15b) et/ou le ressort à lame en résine synthétique renforcée par fibres de carbone (18) sont encastrés dans le noyau du pied (13) et la languette (14) qui sont tous deux en polyuréthane, en particulier en un élastomère de polyuréthane réticulé.

13. Pied artificiel selon l'une des revendications 1 à 8, **caractérisé en ce** que le ressort à lame en résine synthétique renforcée par fibres de carbone (18) est configuré en étant substantiellement plat de la zone du talon à la zone du milieu du pied et que la coudure (11) dans la zone de la cambrure (12) est plus profonde d'au moins 15 mm, de préférence de 16 à 21 mm (distance k).

14. Pied artificiel selon l'une des revendications 1 à 13, **caractérisé en ce** que le ressort à lame en résine synthétique renforcée par fibres de carbone (18) est configuré en étant au moins aussi large dans la zone de la coudure (11) que dans la zone du talon.
